(19) Europäisches Patentamt
European Patent Office
Office européen des brevets

(11) Publication number: 0 269 933 B1

(12) EUROPEAN PATENT SPECIFICATION

(45) Date of publication of patent specification: 30.01.91

(51) Int. Cl.$^5$: C 07 C 43/12, C 07 C 41/06

(21) Application number: 87116812.6

(22) Date of filing: 13.11.87

# (54) Process for preparing 2-bromo-1',2'-dichloroperfluoro diethyl ether.

(30) Priority: 14.11.86 IT 2234886

(43) Date of publication of application:
08.06.88 Bulletin 88/23

(45) Publication of the grant of the patent:
30.01.91 Bulletin 91/05

(84) Designated Contracting States:
BE DE FR GB NL

(56) References cited:
EP-A- 267 626
EP-A-0 120 583
EP-A-0 194 862
EP-A-0 201 871
DE-A-2 554 884
GB-A-2 148 286
US-A-3 586 724

(73) Proprietor: AUSIMONT S.r.l.
Foro Buonaparte 31
I-20121 Milano (IT)

(72) Inventor: Calini, Pierangelo
3, Via Capuana
I-20017 Rho (Milano) (IT)
Inventor: Guglielmo, Gregorio Dr.
7/2, via Fabriano
I-20161 Milano (IT)
Inventor: Guglielmo, Giorgio Dr.
89, via Gramsci
I-30035 Mirano (Venezia) (IT)

(74) Representative: Barz, Peter, Dr. et al
Patentanwälte Dr. V. Schmied-Kowarzik Dipl.-Ing. G. Dannenberg Dr. P. Weinhold Dr. D. Gudel Dipl.-Ing. S. Schubert Dr. P. Barz
Siegfriedstrasse 8
D-8000 München 40 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 269 933 B1

Courier Press, Leamington Spa, England.

## Description

The present invention relates to a process for preparing 2-bromo-1',2'-dichloroperfluoro diethyl ether of the formula:

$$BrCF_2—CF_2—O—CFCl—CF_2Cl \quad (I)$$

This compound is of particular importance as a starting product for the synthesis of bromofluorovinylether via dechlorination in the presence of Zn powder. Said bromofluorovinylether is a comonomer of particular interest for the preparation of fluoroelastomers vulcanizable with peroxides.

It is known to prepare fluorinated ethers by addition of hypofluorite to fluorochloro-olefins, as is described in Italian Patent Application No. 20781 A/85 in the name of the Applicant (EP—A—201871). This kind of reaction is generally conducted under strictly controlled conditions, maintaining the temperature at very low values (from −50 to +50°C), as hypofluorite decomposition reactions can easily occur.

Furthermore, it should be taken into account that the yields can vary in an unforeseeable manner depending on the selected olefin and hypofluorite owing to other secondary reactions.

EP—A—194 862 discloses a process for the preparation of fluoroxy halo compounds which are useful in condensation and/or fluorination reactions with olefins and other organic compounds.

GB—A—2 148 286 discloses a process for producing chlorofluoroethylene telomers which comprises reacting chlorotrifluoroethylene with fluoroxytrifluoromethane or bis-fluoroxydifluoromethane in a chlorofluorohydrocarbon solvent at lower temperatures.

In particular, the reaction of 2-bromo-perfluoroethylhypofluorite with difluoro-dichloroethylene was not known.

The preparation of hypofluorite of formula $BrCF_2—CF_2OF$ is disclosed in Italian Patent Application No. 21632 A/86, which describes the direct reaction of fluorine with $BrCF_2—COF$ on caesium fluoride as a catalyst in the presence of nitrogen as a diluent.

Hypofluorite is an unstable compound and easily leads to β scission reactions; it was observed that by reacting the gas stream of $N_2$-diluted hypofluorite with the olefin dissolved in a solvent, even at low temperature, the product was not formed.

It has unexpectedly been found that the $BrCF_2—CF_2—O—CFCl—CF_2Cl$ adduct is obtained with high yields when the reaction is conducted by condensing the gas stream flowing from the hypofluorite synthesis reactor, by means of cooling and addition, in cocurrent, of a proper fluorinated solvent, which is inert under the reaction conditions, and by feeding the resulting solution into the reactor which contains a $CFCl{=}CFCl$ solution intensely stirred in an identical or analogous fluorine-stable solvent. It is advisable to operate in such manner in order to have always an excess of olefin with respect to hypofluorite.

The reaction product is distilled off from the solvent and the by-products and it is recovered as a fraction having a boiling point of 100—110°C and characterized by mass spectrometry.

The process according to the present invention utilizes, as a reagent, gaseous $BrCF_2—CF_2OF$ in admixture with an inert gas, preferably nitrogen, at a concentration of from 2 to 15% in the gaseous mixture, these being the best conditions for the synthesis of the same hypofluorite.

The solvent which makes possible the condensation of hypofluorite can be admixed to the gaseous stream either as a liquid or as a gas condensable at the temperature at which the addition takes place.

Suitable solvents are the chlorofluorohydrocarbons used as freezing fluids and commercially known under the trade-names Algofrene®, Freon®, etc., and in particular $CF_2Cl_2$, $CF_2Cl—CF_2Cl$.

The reaction temperature ranges from −40 to −150°C. It is apparent that at the lowest temperatures only a few solvents, in particular $CF_2Cl_2$, are suited to the synthesis. The reaction is very quick and, by consequence, also the olefin can be continuously fed, slightly in excess with respect to the stoichiometric amount, to the reactor in which the addition occurs.

The following examples are given to illustrate the present invention.

### Example 1

0.71 Nl/h of $BrCF_2COF$ and 0.7 Nl/h of fluorine diluted in 30 Nl/h of helium were fed to an AISI 316 reactor containing 600 g of a catalyst at 8% of caesium fluoride on copper and thermoregulated at a temperature of 25°C.

The gaseous stream flowing from the reactor, consisting of $BrCF_2—CF_2OF$ and inert gas was mixed with a flow of 7 Nl/h of $CF_2Cl_2$ and conveyed into a cooling coil maintained at a temperature of −78°C in order to cause the condensation of the mixture of hypofluorite and $CF_2Cl_2$.

The liquid mixture so obtained was dropped into an intensely stirred flask containing 29 g of $CFCl{=}CFCl$ (about 0.22 moles) dissolved in 330 g of $CF_2Cl_2$ and maintained, by means of a cooling bath, at a temperature of −78°C.

It was operated for 4 hours, during which 0.1 moles of $BrCF_2—CF_2OF$ were fed.

The resulting reaction mixture was allowed to slowly evaporate and the residue, analyzed by gas chromatography associated with mass spectrography, revealed the presence of a product, which exhibited a mass spectrum (Figure 1) indicative of the structure $BrCF_2—CF_2—O—CFCl—CF_2Cl$.

In fact, among the fragmentations of a compound of formula $BrCF_2—CF_2—O—CFCl—CF_2Cl$ the following are expectable:

a) loss of a chlorine, in alpha position with respect to oxygen, which gives rise to mass fragments 311, 313, 315 respectively (Figure 1). The relative intensities of the peaks observed in the mass spectrum are in a ratio corresponding to the natural isotopic abundance of the remaining chlorine atom or bromine atom;

a) loss of the $BrCF_2$—$CF_2$—O— with formation of fragment —CFCl—$CF_2Cl$. The mass of the latter corresponds to 151 and is present in the mass spectrum along with the signals of mass fragments 153 and 155. The relative intensities are in a ratio corresponding ot the natural isotopic abundance ratio of chlorine.

The illustrated mass spectrum is therefore to be considered as being in accordance with the proposed structure. By distillation of the raw reaction product, unreacted olefin was recovered and a fraction having a boiling point of 100—110°C was then isolated, which was corresponding to the desired product because it was in accordance with the mass spectrum. In order to confirm the structure of the product it has been proved that the same product was obtained by reaction of chlorine with $BrCF_2$—$CF_2$—O—CF=$CF_2$. The yield was of 30% with respect to the fed $CF_2Br$—COF.

Example 2 (comparative test)

The test of Example 1 was repeated, with the exception that the gaseous hypofluorite stream was made to bubble as such into the flask containing CFCl=CFCl in $CFCl_2$—$CF_3$ at a concentration equal to 20% by volume and maintained at a temperature of −78°C.

On analysis, the reaction mixture did not reveal the presence of $BrCF_2$—$CF_2$—O—CFCl—$CF_2Cl$.

## Claims

1. A process for preparing 2-bromo-1′,2′-dichloroperfluoro diethyl ether which comprises reacting, at a temperature of from −40 to −150°C, the bromohypofluorite $BrCF_2CF_2OF$ with the olefin CFCl=CFCl, dissolved in an inert chlorofluorohydrocarbon solvent, said bromohypofluorite being introduced into the reaction mixture in the form of a solution in an inert chlorofluorohydrocarbon solvent.

2. The process of claim 1, wherein said olefin CFCl=CFCl is in excess with respect to the bromohypofluorite.

3. The process of claim 1 or 2, wherein said inert solvent is $CF_2Cl_2$.

## Patentansprüche

1. Verfahren zur Herstellung von 2-Brom-1′,2′-dichlorperfluordiethylether, dadurch gekennzeichnet, daß man das Bromhypofluorit $BrCF_2CF_2OF$ bei einer Temperatur von −40 bis −150°C mit dem Olefin CFCl=CFCl, welches in einem inerten Chlorfluorkohlenwasserstoff-Lösungsmittel gelöst ist, reagieren läßt, wobei das Bromhypofluorit in die Reaktionsmischung in Form einer Lösung in einem inerten Chlorfluorkohlenwasserstoff-Lösungsmittel eingeführt wird.

2. Verfahren nach Anspruch 1, dadurch gekennzeichnet, daß das Olefin CFCl=CFCl in Bezug auf das Bromhypofluorit im Überschuß vorliegt.

3. Verfahren nach Anspruch 1 oder 2, dadurch gekennzeichnet, daß das inerte Lösungsmittel $CF_2Cl_2$ ist.

## Revendications

1. Un procédé de préparation de 2-bromo-1′,2′-dichloroperfluorodiéthyléther qui comprend la réaction, à une température de −40 à −150°C, du bromohypofluorite $BrCF_2CF_2OF$ avec l'oléfine CFCl=CFCl, dissoute dans un solvant chlorofluorohydrocarboné inerte, ledit bromohypofluorite étant introduit dans le mélange réactionnel sous forme d'une solution dans un solvant chlorofluorohydrocarboné inerte.

2. Le procédé selon la revendication 1, dans lequel ladite oléfine CFCl=CFCl est en excès par rapport audit bromohypofluorite.

3. Le procédé selon l'une des revendications 1 ou 2, dans lequel ledit solvant inerte est $CF_2Cl_2$.

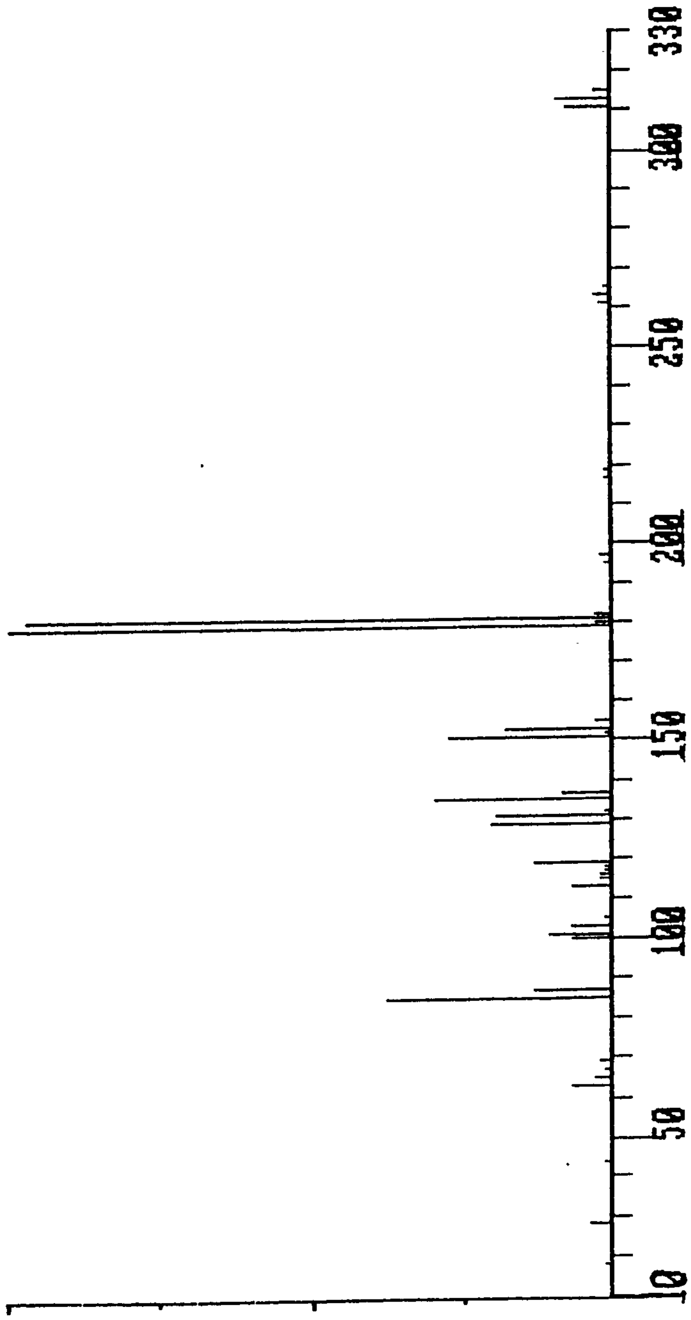


FIG. 1